# EUROPEAN PATENT SPECIFICATION

(11) **EP 4 392 020 B1**
(45) Date of publication and mention of the grant of the patent: **04.06.2025**
(21) Application number: 22765189.0
(22) Date of filing: 29.07.2022
(51) Int. Cl.: A61K 9/127, A61K 47/14, A61K 9/51

(54) **LYOTROPIC LIQUID CRYSTALLINE NANOSYSTEMS WITH ENCAPSULATED BIOACTIVE MACROMOLECULES**
LYOTROPE FLÜSSIGKRISTALLINE NANOSYSTEME MIT VERKAPSELTEN BIOAKTIVEN MAKROMOLEKÜLEN
NANOSYSTÈMES LIQUIDES CRISTALLINS LYOTROPES AVEC MACROMOLÉCULES BIOACTIVES ENCAPSULÉES

(30) Priority: 23.08.2021 GR 20210100567; 08.06.2022 GR 20220100481; 21.07.2022 GR 20220100584
(43) Date of publication of application: 03.07.2024
(73) Proprietor: Uni-Pharma Kleon Tsetis Pharmaceutical Laboratories S.A., Kifisia, Attica 14564 Athens (GR)
(72) Inventor: DEMETZOS, Constantinos, 18757 Piraeus (GR); PISPAS, Asterios, 13123 Athens (GR); CHOUNTOULESI, Maria, 21200 Argos (GR)
(74) Representative: Sonnenhauser, Thomas Martin
(86) International application number: PCT/GR2022/000040
(87) International publication number: WO 2023/026067

(56) References cited:
- WO-A1-2005/046642
- CHUNG H. ET AL.: "Self-assembled 'nanocubicle' as a carrier for peroral insulin delivery", DIABETOLOGIA (2002) 45, 1 January 2002 (2002-01-01), pages 448 - 451, XP055169970, Retrieved from the Internet <URL:http://download.springer.com/static/pdf/42/art%3A10.1007%2Fs00125-001-0751-z.pdf?auth66=1424096256_2a2058a8b6c04c2fb942aa28152c8062&ext=.pdf> [retrieved on 20150216], DOI: 10.1007/s00125-001-0751-z
- ZHAI J. ET AL.: "Non-Lamellar Lyotropic Liquid Crystalline Lipid Nanoparticles for the Next Generation of Nanomedicine", ACS NANO, vol. 13, no. 6, 13 May 2019 (2019-05-13), US, pages 6178 - 6206, XP055937802, ISSN: 1936-0851, Retrieved from the Internet <URL:https://pubs.acs.org/doi/pdf/10.1021/acsnano.8b07961> DOI: 10.1021/acsnano.8b07961
- TRAN N. ET AL.: "Nanostructure and cytotoxicity of self-assembled monoolein-capric acid lyotropic liquid crystalline nanoparticles", RSC ADVANCES, vol. 5, no. 34, 1 January 2015 (2015-01-01), pages 26785 - 26795, XP055972210, Retrieved from the Internet <URL:https://pubs.rsc.org/en/content/articlepdf/2015/ra/c5ra02604k> DOI: 10.1039/C5RA02604K

## Description

The invention relates to compositions of physicochemically stable nanostructured colloidal systems in the form of lyotropic liquid crystals, comprising of amphiphilic lipids, salts of medium-chain fatty acid or their derivatives, copolymers as stabilizers and a bioactive macromolecule comprising peptide bonds.

Oral delivery of bioactive macromolecules, including peptides, proteins and enzymes, gains a continuously increasing interest among the research community, also showing a significant growth and an increasing market share in nowadays. Despite the great progress in this field, there are still important pending issues to overcome as regards the stability of the pharmaceutical forms of these macromolecules and their bioavailability, which in some cases is less than 1%. The development of new technologies such as enteric coating or the use of permeation enhancers or the chemical modification of active substances, has already addressed most of the above-indicated problems. Recently, novel tailored nanoparticles are suggested, which are suitable to encapsulate pharmaceutically active substances and release them through intestinal tract and epithelia.

Lyotropic liquid crystals represent an intermediate state of matter between solid and liquid and they are formed through dispersion and self-assembly of amphiphilic molecules to water. Amphiphilic molecules present different properties between their distinct structural parts (hydrophilic-hydrophobic, polar-nonpolar), and are self-assembled to supramolecular structures with a morphology which is highly dependent from their chemical nature, their concentration and the solvent medium. Other factors that play an important role in the morphology of lyotropic liquid crystals and is directly related to the solvent medium, are the temperature, pH and ionic strength.

The most important mesophases of self-assembled amphiphilic molecules observed, include mono- or bilayer, micellar, cubic, hexagonal or bicontinuous cubic phases.

The scientific community is particularly attracted by the cubic and hexagonal non-bilayer mesophases, as they can form nanoparticulated lyotropic liquid crystal dispersions, suitable to be used as nanocarriers for the delivery of a wide range of active pharmaceutical ingredients. These mesophases present significant advantages, including the ability of controlled release due to the extensive organization of their internal morphology (short range order).

Glyceryl monooleate (GMO) is an amphiphilic, polar, unsaturated monoglyceride being widely used as an emulsifier, also designated as a safe and inert substance by US Food and Drug Administration (FDA). The amphiphilic character of GMO is due to the presence of the hydrophilic glycerol-moiety at the head of the molecule and the hydrophobic hydrocarbon-moiety at the tail of the molecule, thus allowing it to self-assemble in aqueous media forming characteristic, thermodynamically stable liquid crystal structures. The presence of suitably selected surfactants further affects GMO's properties in aqueous solutions, allowing the stabilization of different supramolecular structures, such as cubic and hexagonal, which are ideal for drug entrapment. However, these structures are not physicochemically stable for long time, due to the hydrolysis of the ester bond of the GMO, which breaks the hydrophobic hydrocarbon chain from the hydrophilic glycerol, furthermore due to the double bond oxidation of the hydrocarbon chain.

Amphiphilic polymers represent another class of molecules widely used in several pharmaceutical forms, not only as emulsion stabilizers but also as viscosity regulators. Amphiphilic polymers, like all other amphiphilic molecules, are self-organized to various characteristic structures depending on the dispersion medium. A special category of this family consists of amphiphilic triblock copolymers, which although they show great complexity, at the same time, they are formed in a variety of morphologies particularly useful in various applications. Triblock copolymer poly(ethylene oxide)-b-poly(propylene oxide)-b-poly(ethylene oxide) (PEO-b-PPO-b-PEO), also known as Poloxamer, has been reported for the stabilization of cubic GMO structures in aqueous media (M. Chountoulesi, et al. Cubic lyotropic liquid crystals as drug delivery carriers: Physicochemical and morphological studies. International Journal of Pharmaceutics 2018, 550, 57-70).

Medium chain fatty acids, especially those carrying between 6 and 12 carbon atoms in the aliphatic lipid chain, and their derivatives are commonly used as permeation enhancers to increase the bioavailability of certain orally administered pharmaceutical substances. The sodium salt of decanoic acid (C10) and the sodium salt of 8-((2-hydroxybenzoyl)amino) octanoic acid (Salcaprozate sodium - SNAC) represent two characteristic candidates of this family and numeral clinical studies reported as regards their safety and stability towards this direction (Twarog, C.; et al. Intestinal Permeation Enhancers for Oral Delivery of Macromolecules: A Comparison between Salcaprozate Sodium (SNAC) and Sodium Caprate (C10). Pharmaceutics 2019, 11, 78).

The influence of decanoic acid on the self-assembly of GMO mesophases in aqueous media is already reported (N. Tran et al. Nanostructure and cytotoxicity of self-assembled monoolein-capric acid lyotropic liquid crystalline nanoparticles RSC Adv., 2015, 5, 26785-26795). Another report relates to GMO/poly(ethylene oxide)-b-poly(propylene oxide)-b-poly(ethylene oxide) cubic nanoparticles as potential drug delivery system of the poorly water-soluble active substance simvastatin (J. Lai, et al. Glyceryl Monooleate/Poloxamer 407 Cubic Nanoparticles as Oral Drug Delivery Systems: I. In Vitro Evaluation and Enhanced Oral Bioavailability of the Poorly Water-Soluble Drug Simvastatin AAPS Pharm Sci Tech, 2009, 10, 960).

Insulin is a peptide hormone produced by β-cells in the pancreas. It plays a dominant role in the metabolism of carbohydrates, fats and proteins in the human body, and most importantly, in the regulation of blood glucose levels. Insulin deficiency is the cause of diabetes mellitus. SNAC-coated insulin microspheres have been developed to protect the drug substance from enzyme degradation while at the same time these microspheres exhibit increased permeability from the gastrointestinal tract [R. Ql and Q. N. Ping, Gastrointestinal absorption enhancement of insulin by administration of enteric microspheres and SNAC to rats. J. Microencapsulation, 2004 (21) 37-45]. Another report relates to insulin nanocubicles stabilized by GMO and non-ionic copolymer Pluronic F-127 (Poloxamer 407) in a mixture of ethanol and propyleneglycol [H. Chung, et al. Self-assembled "nanocubicle" as a carrier for peroral insulin delivery. Diabetologia 2002 (45) 448-451]. Several nanocarrier formulations for insulin delivery related to chitosan coated nanoparticles, PLGA-insulin nanoparticles, dextran-insulin nanoparticles, and solid lipid-insulin nanoparticles are reported in the recent literature [Sharma et al. Nanoparticle based insulin delivery system: the next generation efficient therapy for Type 1 diabetes J Nanobiotechnol2015 (13), 74 (13 pages)].

Document EP2331072 relates to oral compositions comprising a protein, an absorption enhancer, and a protease inhibitor.

The present invention relates to compositions of physicochemically stable colloid systems in the form of lyotropic liquid crystals comprising glycerol monooleate, a salt of C6-C12 fatty acid or its derivative with an aromatic substituent, an amphiphilic block copolymer with the following chemical formula H(OCH₂CH₂)ₐ(OCH(CH₃)CH₂)_{b}(OCH₂CH₂)ₐOH, wherein 2≤a≤150 and 15≤b≤70 and a bioactive macromolecule with peptide bonds.

It was surprisingly found that the lyotropic liquid crystalline nanoparticles of the present invention, present a nanocompartmentalized internal structure, which although is consistent, at the same time, is highly loose, allowing it to be highly flexible to encapsulate a variety of bioactive macromolecules with peptide bonds of different structures and different molecular weights, without affecting their physicochemical characteristics overall.

Furthermore, it was surprisingly found that the self-assembled nanoparticles of the present invention, in the form of lyotropic liquid crystals carrying encapsulated bioactive macromolecules having peptide bonds, form time-stable colloidal nanosystems in terms of size, size distribution and ζ-potential, regardless of their internal structure organization after the encapsulation of bioactive macromolecules with peptide bonds.

In addition, the self-assembled nanoparticles of the present invention, in the form of lyotropic liquid crystals, carrying encapsulated bioactive macromolecules having peptide bonds, remain physicochemically stable colloidal nanosystems over time, in both simulated gastric fluid (pH = 1.6), and in simulated intestinal fluid (pH = 6.5).

According to the present invention, the self-assembled nanoparticles with encapsulated bioactive macromolecules having peptide bonds, have an average diameter ranging from 50 nm to 900 nm, preferably between 100 nm and 500 nm, more preferably between 100 nm and 300 nm.

According to the present invention, the concentration of glycerol monooleate in the composition of lyotropic liquid crystalline nanoparticles, ranges from 10 mg/mL to 100 mg/mL, preferably ranges from 20 mg/mL to 80 mg/mL, more preferably is 20 mg/mL.

According to the present invention, the salt of medium-chain fatty acid used for the development of the lyotropic liquid crystalline nanoparticles, is selected among the alkali salts of fatty acids having from 6 to 12 carbon atoms in their aliphatic chain or their derivatives with an aromatic substituent.

According to the present invention, the salt of the C6-C12 fatty acid used for the development of lyotropic liquid crystalline nanoparticles, is selected between the alkaline salts octanoic, decanoic or dodecanoic acids and its derivative is selected among the alkaline salts of 8-((2-hydroxybenzoyl)amino)octanoic acid.

In a preferred embodiment, the salt of the C6-C12 fatty acid or its derivative with an aromatic substituent, used for the development of lyotropic liquid crystalline nanoparticles of the present invention, is the sodium salt of octanoic acid.

In a preferred embodiment, the salt of the C6-C12 fatty acid or its derivative with an aromatic substituent, used for the development of lyotropic liquid crystalline nanoparticles of the present invention, is the sodium salt of decanoic acid.

In a preferred embodiment, the salt of the C6-C12 fatty acid or its derivative with an aromatic substituent, used for the development of lyotropic liquid crystalline nanoparticles of the present invention, is the sodium salt of dodecanoic acid.

In a preferred embodiment, the salt of the C6-C12 fatty acid or its derivative with an aromatic substituent, used for the development of lyotropic liquid crystalline nanoparticles of the present invention, is the sodium salt of 8-((2-hydroxybenzoyl)amino)octanoic acid.

According to the present invention, the amphiphilic block copolymer for the development of lyotropic liquid crystalline nanoparticles, has the following chemical formula H(OCH₂CH₂)ₐ(OCH(CH₃)CH₂)_{b}(OCH₂CH₂)ₐOH, wherein, 2≤a≤150 and 15≤b≤70, preferably is selected among those wherein 60≤a≤150 and 25≤b≤70, more preferably is selected among those wherein 95≤a≤105 and 55≤b≤70.

In a preferred embodiment, the amphiphilic block copolymer for the development of lyotropic liquid crystalline nanoparticles, has the chemical formula H(OCH₂CH₂)₈₀(OCH(CH₃)CH₂)₂₇(OCH₂CH₂)₈₀OH.

In a preferred embodiment, the amphiphilic block copolymer for the development of lyotropic liquid crystalline nanoparticles, has the chemical formula H(OCH₂CH₂)₆₄(OCH(CH₃)CH₂)₃₇(OCH₂CH₂)₆₄OH.

In a preferred embodiment, the amphiphilic block copolymer for the development of lyotropic liquid crystalline nanoparticles, has the chemical formula H(OCH₂CH₂)₁₄₁(OCH(CH₃)CH₂)₄₄(OCH₂CH₂)₁₄₁OH.

In a preferred embodiment, the amphiphilic block copolymer for the development of lyotropic liquid crystalline nanoparticles, has the chemical formula H(OCH₂CH₂)₁₀₁(OCH(CH₃)CH₂)₅₆(OCH₂CH₂)₁₀₁OH

In a preferred embodiment, the amphiphilic block copolymer for the development of lyotropic liquid crystalline nanoparticles, has the chemical formula H(OCH₂CH₂)₉₈(OCH(CH₃)CH₂)₆₇(OCH₂CH₂)₉₈OH

According to the present invention, the weight ratio in milligrams (mg) of glycerol monooleate to the salt of C6-C12 fatty acid or its derivative with an aromatic substituent, to the amphiphilic block copolymer, per milliliter (mL) in the final composition, is 20:A:B, wherein 1≤ A ≤10 and 1≤ B ≤10, preferably 2≤ A ≤4 and 3≤ B ≤7.

In a preferred embodiment, the weight ratio in milligrams (mg) of glycerol monooleate to the salt of C6-C12 fatty acid or its derivative with an aromatic substituent, to the amphiphilic block copolymer, per milliliter (mL) in the final composition, is 20:2:5.

In a preferred embodiment, the weight ratio in milligrams (mg) of glycerol monooleate to the salt of C6-C12 fatty acid or its derivative with an aromatic substituent, to the amphiphilic block copolymer, per milliliter (mL) in the final composition, is 20:4:5.

In a preferred embodiment, the lyotropic liquid crystalline nanoparticles in the composition of the present invention consist of glycerol monooleate, the sodium salt of octanoic acid and the copolymer H(OCH₂CH₂)ₐ(OCH(CH₃)CH₂)_{b}(OCH₂CH₂)ₐOH, wherein 95≤a≤105 and 55≤b≤70 in a weight ratio 20:2:5 per mL of the final composition solution.

In a more preferred embodiment, the lyotropic liquid crystalline nanoparticles in the composition of the present invention consist of glycerol monooleate, the sodium salt of octanoic acid and the copolymer H(OCH₂CH₂)₉₈(OCH(CH₃)CH₂)₆₇(OCH₂CH₂)₉₈OH in a weight ratio 20:2:5 per mL of the final composition solution.

In a preferred embodiment, the lyotropic liquid crystalline nanoparticles in the composition of the present invention consist of glycerol monooleate, the sodium salt of octanoic acid and the copolymer H(OCH₂CH₂)ₐ(OCH(CH₃)CH₂)_{b}(OCH₂CH₂)ₐOH, wherein 95≤a≤105 and 55≤b≤70 in a weight ratio 20:4:5 per mL of the final composition solution.

In a more preferred embodiment, the lyotropic liquid crystalline nanoparticles in the composition of the present invention consist of glycerol monooleate, the sodium salt of octanoic acid and the copolymer H(OCH₂CH₂)₉₈(OCH(CH₃)CH₂)₆₇(OCH₂CH₂)₉₈OH in a weight ratio 20:4:5 per mL of the final composition solution.

In a preferred embodiment, the lyotropic liquid crystalline nanoparticles in the composition of the present invention consist of glycerol monooleate, the sodium salt of 8-((2-hydroxybenzoyl)amino)octanoic acid and the copolymer H(OCH₂CH₂)ₐ(OCH(CH₃)CH₂)_{b}(OCH₂CH₂)ₐOH, wherein 95≤a≤105 and 55≤b≤70 in a weight ratio 20:2:5 per mL of the final composition solution.

In a more preferred embodiment, the lyotropic liquid crystalline nanoparticles in the composition of the present invention consist of glycerol monooleate, the sodium salt of 8-((2-hydroxybenzoyl)amino)octanoic acid and the copolymer H(OCH₂CH₂)₉₈(OCH(CH₃)CH₂)₆₇(OCH₂CH₂)₉₈OH in a weight ratio 20:2:5 per mL of the final composition solution.

In a preferred embodiment, the lyotropic liquid crystalline nanoparticles in the composition of the present invention consist of glycerol monooleate, the sodium salt of 8-((2-hydroxybenzoyl)amino)octanoic acid and the copolymer H(OCH₂CH₂)ₐ(OCH(CH₃)CH₂)_{b}(OCH₂CH₂)ₐOH, wherein 95≤a≤105 and 55≤b≤70 in a weight ratio 20:4:5 per mL of the final composition solution.

In a more preferred embodiment, the lyotropic liquid crystalline nanoparticles in the composition of the present invention consist of glycerol monooleate, the sodium salt of 8-((2-hydroxybenzoyl)amino)octanoic acid and the copolymer H(OCH₂CH₂)₉₈(OCH(CH₃)CH₂)₆₇(OCH₂CH₂)₉₈OH in a weight ratio 20:4:5 per mL of the final composition solution.

According to the present invention, the bioactive macromolecule having peptide bonds in the composition of lyotropic liquid crystalline nanoparticles is selected between enzymes, proteins and hormones.

In a preferred embodiment, the bioactive macromolecule having peptide bonds in the composition of lyotropic liquid crystalline nanoparticles is the lysozyme enzyme.

In a preferred embodiment, the bioactive macromolecule having peptide bonds in the composition of lyotropic liquid crystalline nanoparticles is the bovine serum albumin protein (BSA).

In a preferred embodiment, the bioactive macromolecule having peptide bonds in the composition of lyotropic liquid crystalline nanoparticles is insulin hormone.

In a preferred embodiment, the bioactive macromolecule having peptide bonds in the composition of lyotropic liquid crystalline nanoparticles is calcitonin hormone.

In a preferred embodiment, the bioactive macromolecule having peptide bonds in the composition of lyotropic liquid crystalline nanoparticles is the octapeptide octreotide.

In a preferred embodiment, the bioactive macromolecule having peptide bonds in the composition of lyotropic liquid crystalline nanoparticles is a glucagon-like peptide 1 (GLP-1) analogue, preferably liraglutide, semaglutide or dulaglutide.

According to the present invention, the concentration of the bioactive macromolecule having peptide bonds in the composition of lyotropic liquid crystalline nanoparticles is between 0.01 mg/mL and 10.0 mg/mL in the final composition, preferably between 0.1 mg/mL and 5.0 mg/mL in the final composition.

In a preferred embodiment, the concentration of the bioactive macromolecule having peptide bonds in the composition of lyotropic liquid crystalline nanoparticles is 0.5 mg/mL.

In a preferred embodiment, the concentration of the bioactive macromolecule having peptide bonds in the composition of lyotropic liquid crystalline nanoparticles is 1.0 mg/mL.

According to the present invention, the weight ratio of glycerol monooleate to the salt of C6-C12 fatty acid or its derivative with an aromatic substituent, to the amphiphilic block copolymer, to the bioactive macromolecule having peptide bonds, in the final composition, is 20:A:B:C, wherein 1≤ A ≤10, 1≤ B ≤10 and 0.01≤ C ≤10, preferably wherein 2≤ A ≤4, 3≤ B ≤7 and 0.1≤ C ≤5, more preferably is 20:2:5:0.5 or 20:2:5:1 or 20:4:5:0.5 or 20:4:5:1.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and insulin, in a weight ratio of 20:2:5:0.5 per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and insulin, in a weight ratio of 20:4:5:1.0 per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and insulin, in a weight ratio of 20:2:5:0.5 per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and insulin, in a weight ratio of 20:4:5:1.0 per mL of the final composition solution.

It was found that the composition of the current invention consisting of glycerol monooleate, the sodium salt of octanoic acid, the block copolymer H(OCH₂CH₂)₉₈(OCH(CH₃)CH₂)₆₇(OCH₂CH₂)₉₈OH and insulin in a weight ratio of 20:2:5:0.5 per mL of the final composition solution, presents an unexpectantly high insulin entrapment in a weight percentage of more than 99% and significantly improved physicochemical stability in simulated gastric and intestinal environment conditions.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and bovine serum albumin, in a weight ratio of 20:2:5:0.5 per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and bovine serum albumin, in a weight ratio of 20:2:5:1.0 per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and bovine serum albumin, in a weight ratio of 20:2:5:0.5 per mL of the final composition solution.

It was found that the composition of the current invention consisting of glycerol monooleate, the sodium salt of 8-((2-hydroxybenzoyl)amino)octanoic acid, the block copolymer H(OCH₂CH₂)₉₈(OCH(CH₃)CH₂)₆₇(OCH₂CH₂)₉₈OH and bovine serum albumin in a weight ratio of 20:2:5:0.5 per mL of the final composition solution, presents significantly improved physicochemical stability in simulated gastric and intestinal environment conditions.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and bovine serum albumin, in a weight ratio of 20:2:5:1.0 per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and lysozyme, in a weight ratio of 20:2:5:0.5 per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and lysozyme, in a weight ratio of 20:2:5:1.0 per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and lysozyme, in a weight ratio of 20:2:5:0.5 per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and lysozyme, in a weight ratio of 20:2:5:1.0 per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and octreotide, in a weight ratio of 20:A:B:0.5, wherein 1≤ A ≤10, and 1≤ B ≤10, per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and octreotide, in a weight ratio of 20:A:B:0.5, wherein 1≤ A ≤10, and 1≤ B ≤10, per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and a GLP-1 analogue, in a weight ratio of 20:A:B:C, wherein 1≤ A ≤10, 1≤ B ≤10 and 0.01≤ C ≤10, preferably wherein 2≤ A ≤4, 3≤ B ≤7 and 0.5≤ C ≤6, per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and a GLP-1 analogue, in a weight ratio of 20:A:B:C, wherein 1≤ A ≤10, 1≤ B ≤10 and 0.01≤ C ≤10, preferably wherein 2≤ A ≤4, 3≤ B ≤7 and 0.5≤ C ≤6, per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105and 55≤b≤70 and liraglutide, in a weight ratio of 20:A:B:6, wherein 1≤ A ≤10, and 1≤ B ≤10, per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and liraglutide, in a weight ratio of 20:A:B:6, wherein 1≤ A ≤10, and 1≤ B ≤10, per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and semaglutide, in a weight ratio of 20:A:B:1, wherein 1≤ A ≤10, and 1≤ B ≤10, per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and semaglutide, in a weight ratio of 20:A:B:1, wherein 1≤ A ≤10, and 1≤ B ≤10, per mL of the final composition solution.

In a preferred embodiment, the composition of the present invention consists of glycerol monooleate, the sodium salt of octanoic acid, the block copolymer H(OCH₂CH₂)ₐ(OCH(CH₃)CH₂)_{b}(OCH₂CH₂)ₐOH, wherein 95≤a≤105 and 55≤b≤70 and dulaglutide, in a weight ratio of 20:A:B:C, wherein 1≤ A ≤10, 1≤ B ≤10 and 0.5≤ C ≤5, per mL of the final composition solution.

In a preferred embodiment, the composition of the present invention consists of glycerol monooleate, the sodium salt of 8-((2-hydroxybenzoyl)amino)octanoic acid, the block copolymer H(OCH₂CH₂)ₐ(OCH(CH₃)CH₂)_{b}(OCH₂CH₂)ₐOH, wherein 95≤a≤105 and 55≤b≤70 and dulaglutide, in a weight ratio of 20:A:B:C, wherein 1≤ A ≤10, 1≤ B ≤10 and 0.5≤ C ≤5, per mL of the final composition solution.

According to the present invention, the composition in the form of lyotropic liquid crystalline nanoparticles with encapsulated bioactive macromolecules having peptide bonds, is formulated with pharmaceutically acceptable excipients to be suitable for oral, intranasal, subcutaneous, intraperitoneal, intravenous or topical administration.

In a preferred embodiment, the composition in the form of lyotropic liquid crystalline nanoparticles with encapsulated bioactive macromolecules having peptide bonds, is formulated with pharmaceutically acceptable excipients to be suitable for oral administration.

In a preferred embodiment, the composition in the form of lyotropic liquid crystalline nanoparticles with encapsulated bioactive macromolecules having peptide bonds, for oral administration, may be further coated with pH-responsive polymers which allow sustained release in the desired environment.

In a preferred embodiment, the composition in the form of lyotropic liquid crystalline nanoparticles with encapsulated bioactive macromolecules having peptide bonds, for oral administration, may be coated with synthetic acrylic and methacrylic polymers suitable for pH-dependent release.

In a preferred embodiment, the composition in the form of lyotropic liquid crystalline nanoparticles with encapsulated bioactive macromolecules having peptide bonds, for oral administration, is coated with methacrylic acid-ethyl acrylate copolymer (1:1) for targeted release in the duodenum at pH values above than 5.5.

In a preferred embodiment, the composition in the form of lyotropic liquid crystalline nanoparticles with encapsulated bioactive macromolecules having peptide bonds, for oral administration, is coated with methacrylic acid-methyl methacrylate copolymer (1:1) for targeted release in the medium of the small intestine at pH values above than 6.0.

In a preferred embodiment, the composition in the form of lyotropic liquid crystalline nanoparticles with encapsulated bioactive macromolecules having peptide bonds, for oral administration, is coated with methacrylic acid-methyl methacrylate copolymer (1:2) for targeted release in the small intestine at pH values above than 7.0.

In a preferred embodiment, the composition in the form of lyotropic liquid crystalline nanoparticles with encapsulated bioactive macromolecules having peptide bonds, for oral administration, is coated with the high permeability ethyl acrylate-methyl methacrylate-trimethylaminoethyl methacrylate chloride copolymer (1:2:0.2) for sustained release.

The entrapment of bioactive macromolecules in the lyotropic liquid crystalline nanosystems is evaluated using the BCA Protein Assay for the colorimetric detection and quantitation of total protein, showing an entrapment efficiency of more than 85% to all studied nanosystems.

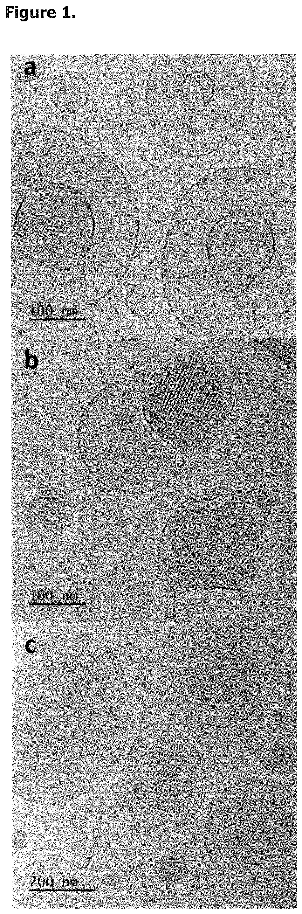

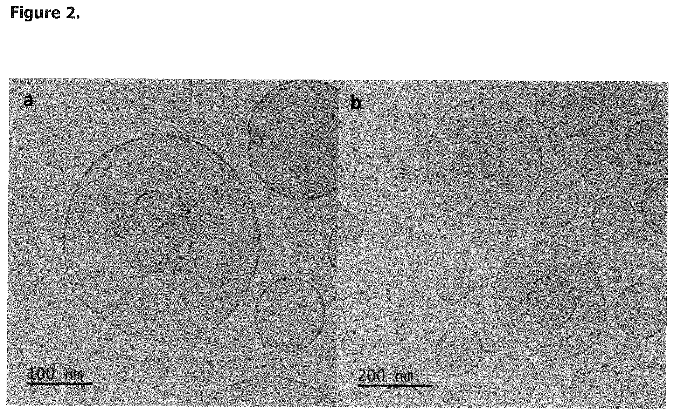

The present invention is further described with the following indicative, non-limiting examples, and figures 1 and 2, where:
Figure 1 shows cryogenic transmission electron microscopy (cryo-TEM) images of (a) the empty nanocarrier and (b) and (c) the insulin-entrapped lyotropic liquid crystalline nanosystems of Example 1 and
Figure 2 shows cryo-TEM images of the bovine serum albumin-encapsulated lyotropic liquid crystalline nanosystems of Example 2.

**Example 1.** Composition in the form of lyotropic liquid crystalline nanoparticles consisting of glyceryl monooleate, the sodium salt of 8-((2-hydroxybenzoyl)amino)octanoic acid, H(OCH₂CH₂)₉₈(OCH(CH₃)CH₂)₆₇(OCH₂CH₂)₉₈OH copolymer and insulin, in a weight ratio 20:2:5:0.5 per mL of the final composition.

A 20 mg/mL glycerol monooleate solution in purified water is prepared under mild heating (50° C). An aqueous solution of the sodium salt of 8-((2-hydroxybenzoyl)amino)octanoic acid, followed by an aqueous solution of H(OCH₂CH₂)₉₈(OCH(CH₃)CH₂)₆₇(OCH₂CH₂)₉₈OH copolymer, followed by an aqueous solution of insulin are added in the solution of glyceryl monoleate, so that the weight ratio of the components in the final composition is 20:2:5:0.5 mg per mL. The mixture is heated at 50° C for 5 minutes, then it was sonicated in a bath sonicator, until a milky dispersion was formed, further followed by followed by two 1-min sonication cycles (amplitude 70, cycle 0,7), and interrupted by a 1-min resting period, using a probe sonicator. The resultant dispersion is allowed to anneal for 30 min. The final dispersion is opaque white and stored at room temperature.

The average hydrodynamic diameter (Dₕ) representing the particle size and the particle size distribution (Polydispersity Index - PDI) of the nanoparticles in the composition of Example 1 is determined by the Dynamic Light Scattering (DLS) technique. The ζ-potential of the nanoparticles is determined by the Electrophoretic Light Scattering (ELS) technique. The physicochemical and morphological characteristics of the nanoparticles immediately after their preparation are summarized in Table 1.

**Table 1. Average hydrodynamic diameter (Dₕ), Polydispersity Index (PDI) and ζ-potential of the compositions of Example 1**

| **Parameter** | **Empty Nanocarrier** | **Composition of Example 1** |
|---|---|---|
| Dₕ (nm) | 136.0 ± 0.6 | 128.0 ± 2.2 |
| PDI | 0.310 ± 0.011 | 0.440 ± 0.060 |
| ζ-potential (mV) | -10.0 ± 0.5 | -42.0 ± 0.3 |

The colloidal suspensions of the nanoparticles of the present invention show remarkable physicochemical stability over time in both their particle size and their size distribution.

The morphology of the compositions of Example 1 was studied and qualitatively evaluated by cryogenic transmission electron microscopy (cryo-TEM). As regards the empty nanosystem, which does not contain the encapsulated insulin (Figure 1a), it was observed a repeated characteristic liquid crystalline unique morphology of a double-compartment nanostructure, where an inner, irregular, perforated porous-like structure is enclosed in an outer vesicle, representing a bubble. The whole morphology tends to donut-like morphology, indicating a modulated liquid crystalline mesophase, which appeared for the first time in the prior art. The unique morphology resembles also to the cell with its nucleus inside enclosed by the porous nuclear membrane/envelop. The empty nanostructures of Example 1 exhibited sizes of 160-900 nm and membrane thickness of 3-4 nm. The presence of the dispersed, crowded vesicular structures causes a further stabilization of the liquid crystalline nanoparticles by forming a "coating" layer of material, covering the ordered nanoparticles, helping to keep them dispersed in aqueous solution. The bubble-like outer vesicle exhibits a protective role over the inner irregular nanocompartmentalized core.

The incorporation of insulin (Figure 1b and 1c) yielded higher grade of internal organization. The lyotropic liquid crystalline nanoparticles with the entrapped insulin presented confined nanoparticles with a regularly ordered internal structure along with liquid crystalline nanoparticles with lower grade of internal organization (Figure 1b). These less periodical, irregular structures tend to resemble "sponge"-like structures (*L3* mesophase) consist of an outer layer built of intersecting lamellas and an apparent dense inner core with highly disordered interior and with absence of long-range order and periodicity. The nanostructures of Example 1 with entrapped insulin present sizes of 40 -220 nm, furthermore, are co-existing with a prevailing population of small, spherical vesicles with no inner structure, with sizes 15-330 nm. The lyotropic liquid crystalline nanostructures with entrapped insulin are also co-existing with perforated porous-like structures of many inner intersecting layers and an apparent dense inner core with highly disordered interior (Figure 1c). Their total sizes are in the range of 130-550 nm, with a membrane thickness of 3-4 nm.

**Example 2.** Composition in the form of lyotropic liquid crystalline nanoparticles consisting of glyceryl monooleate, the sodium salt of 8-((2-hydroxybenzoyl)amino)octanoic acid, H(OCH₂CH₂)₉₈(OCH(CH₃)CH₂)₆₇(OCH₂CH₂)₉₈OH copolymer and bovine serum albumin, in a weight ratio 20:2:5:0.5 per mL of the final composition.

A 20 mg/mL glycerol monooleate solution in purified water is prepared under mild heating (50° C). An aqueous solution of the sodium salt of 8-((2-hydroxybenzoyl)amino)octanoic acid, followed by an aqueous solution of H(OCH₂CH₂)₉₈(OCH(CH₃)CH₂)₆₇(OCH₂CH₂)₉₈OH copolymer, followed by an aqueous solution of bovine serum albumin were added in the solution of glyceryl monooleate, in order to prepare two new nanosystems in which the weight ratio of the components in the final composition is 20:2:5:0.5 mg per mL and 20:2:5:1.0, respectively. The mixture is heated at 50° C for 5 minutes, then it was sonicated in a bath sonicator, until a milky dispersion was formed, further followed by two 1-min sonication cycles (amplitude 70, cycle 0,7), and interrupted by a 1-min resting period, using a probe sonicator. The resultant dispersions are allowed to anneal for 30 min. The final dispersions are opaque white and stored at room temperature.

The average hydrodynamic diameter (Dₕ) representing the particle size and the particle size distribution (Polydispersity Index - PDI) of the nanoparticles in the composition of Example 2 is determined by the Dynamic Light Scattering (DLS) technique. The *ζ*-potential of the nanoparticles is determined by the Electrophoretic Light Scattering (ELS) technique. The physicochemical and morphological characteristics of the nanoparticles immediately after their preparation are summarized in Table 2.

**Table 2. Average hydrodynamic diameter (Dₕ), Polydispersity Index (PDI) and ζ-potential of the compositions of Example 2**

| **Parameter** | **Empty Nanocarrier** | **Composition of Example 2 with a weight ratio 20:2:5:0.5 mg / mL** | **Composition of Example 2 with a weight ratio 20:2:5:1.0 mg / mL** |
|---|---|---|---|
| Dₕ (nm) | 138.0 ± 0.6 | 160.0 ± 6.5 | 176.0 ± 4.0 |
| PDI | 0.320 ± 0.011 | 0.480 ± 0.014 | 0.480 ± 0.018 |
| ζ-potential (mV) | -10.0 ± 0.5 | -36.0 ± 1.0 | -45.0 ± 2.0 |

The colloidal suspensions of the nanoparticles of the present invention show remarkable physicochemical stability over time in both their particle size and their size distribution.

The morphology of the compositions of Example 2 was studied and qualitatively evaluated by cryogenic transmission electron microscopy (cryo-TEM). In the presence of bovine serum albumin (Figure 2), no significant changes were observed in the morphological behavior, in comparison to the corresponding blank nanosystem (Figure 1a). Briefly, the characteristic double-compartment nanostructure, with an inner, irregular, perforated porous-like structure being enclosed in an outer vesicle is disclosed again (Figure 2a). The vesicles containing irregular structures inside, exhibiting sizes of 200-800 nm and membrane thickness of 3-4 nm, while the population of the empty vesicles had sizes at 10-600 nm and membrane thickness of 3-4 nm (Figure 2b).

## Claims

1. A composition in the form of lyotropic liquid crystalline nanoparticles comprising glycerol monooleate, a salt of a C6-C12 fatty acid or its derivative with an aromatic substituent, an amphiphilic block copolymer with a chemical formula of H(OCH₂CH₂)ₐ(OCH(CH₃)CH₂)_{b}(OCH₂CH₂)ₐOH, wherein 2≤a≤150 and 15≤b≤70, and a bioactive macromolecule having peptide bonds.

2. The composition according to claim 1, wherein the salt of the C6-C12 fatty acid is selected between the alkaline salts of octanoic, decanoic or dodecanoic acid and its derivative is selected between the alkaline salts of the 8-((2-hydroxybenzoyl)amino)octanoic acid, preferably the sodium salt of octanoic acid and 8-((2-hydroxybenzoyl)amino)octanoic acid, more preferably is the sodium salt of 8-((2-hydroxybenzoyl)amino)octanoic acid.

3. The composition according to any of claims 1 and 2, wherein the amphiphilic block copolymer with the chemical formula H(OCH₂CH₂)ₐ(OCH(CH₃)CH₂)_{b}(OCH₂CH₂)ₐOH, wherein, 2≤a≤150 and 15≤b≤70, preferably is selected among those wherein 60≤a≤150 and 25≤b≤70, more preferably is selected among those wherein 95≤a≤105and 55≤b≤70.

4. The composition according to any of claims 1 to 3, wherein the bioactive macromolecule having peptide bonds is selected between enzymes, proteins and hormones, preferably between lysozyme, bovine serum albumin, insulin, calcitonin, octreotide, liraglutide, semaglutide, and dulaglutide.

5. The composition according to any of claims 1 to 4, wherein the concentration of glycerol monooleate in the composition, is from 10 mg/mL to 100 mg/mL, preferably from 20 mg/mL to 80 mg/mL, more preferably is 20 mg/mL.

6. The composition according to any of claims 1 to 5, wherein the weight ratio of glycerol monooleate to the salt of C6-C12 fatty acid or its derivative with an aromatic substituent, to the amphiphilic block copolymer, in the final composition, is 20:A:B, wherein 1≤ A ≤10 and 1≤ B ≤10, preferably 2≤ A ≤4 and 3≤ B ≤7, more preferably is 20:2:5 or 20:4:5.

7. The composition according to any of claims 1 to 6, wherein the concentration of the bioactive macromolecule having peptide bonds is between 0.01 mg/mL and 10.0 mg/mL in the final composition, preferably between 0.1 mg/mL and 5.0 mg/mL in the final composition.

8. The composition according to any of claims 1 to 7, wherein the weight ratio of glycerol monooleate to the salt of C6-C12 fatty acid or its derivative with an aromatic substituent, to the amphiphilic block copolymer, to the bioactive macromolecule having peptide bonds, in the final composition, is 20:A:B:C, wherein 1≤ A ≤10, 1≤ B ≤10 and 0.01≤ C ≤10, preferably wherein 2≤ A ≤4, 3≤ B ≤7 and 0.1≤ C ≤5, more preferably is 20:2:5:0.5 or 20:2:5:1 or 20:4:5:0.5 or 20:4:5:1.

9. The composition according to any of claims 1 to 8, which is formulated with pharmaceutically acceptable excipients for oral, intranasal, subcutaneous, intraperitoneal, intravenous or topical administration, preferably for oral administration.

## Patentansprüche

1. Zusammensetzung in Form von lyotropen flüssigkristallinen Nanopartikeln, umfassend Glycerinmonooleat, ein Salz einer C6-C12-Fettsäure oder deren Derivat mit einem aromatischen Substituenten, ein amphiphiles Blockcopolymer mit der chemischen Formel H(OCH₂CH₂)ₐ(OCH(CH₃)CH₂)_{b}(OCH₂CH₂)ₐOH, wobei 2 ≤ a ≤ 150 und 15 ≤ b ≤ 70, und ein bioaktives Makromolekül mit Peptidbindungen.

2. Zusammensetzung nach Anspruch 1, wobei das Salz der C6-C12-Fettsäure ausgewählt ist aus den alkalischen Salzen von Octansäure, Decansäure oder Dodecansäure und deren Derivat ausgewählt ist aus den alkalischen Salzen der 8-((2-Hydroxybenzoyl)amino)octansäure, vorzugsweise dem Natriumsalz von Octansäure und 8-((2-Hydroxybenzoyl)amino)octansäure, und noch bevorzugter das Natriumsalz von 8-((2-Hydroxybenzoyl)amino)octansäure ist.

3. Zusammensetzung nach Anspruch 1 oder 2, wobei das amphiphile Blockcopolymer mit der chemischen Formel H(OCH₂CH₂)ₐ(OCH(CH₃)CH₂)_{b}(OCH₂CH₂)ₐOH, wobei 2 ≤ a ≤ 150 und 15 ≤ b ≤ 70, vorzugsweise ausgewählt ist aus solchen, bei denen 60 ≤ a ≤ 150 und 25 ≤ b ≤ 70, noch bevorzugter ausgewählt ist aus solchen, bei denen 95 ≤ a ≤ 105 und 55 ≤ b ≤ 70.

4. Zusammensetzung nach einem der Ansprüche 1 bis 3, wobei das bioaktive Makromolekül mit Peptidbindungen ausgewählt ist aus Enzymen, Proteinen und Hormonen, vorzugsweise aus Lysozym, Rinderserumalbumin, Insulin, Calcitonin, Octreotid, Liraglutid, Semaglutid und Dulaglutid.

5. Zusammensetzung nach einem der Ansprüche 1 bis 4, wobei die Konzentration von Glycerinmonooleat in der Zusammensetzung 10 mg/ml bis 100 mg/ml, vorzugsweise 20 mg/ml bis 80 mg/ml, noch bevorzugter 20 mg/ml beträgt.

6. Zusammensetzung nach einem der Ansprüche 1 bis 5, wobei das Gewichtsverhältnis von Glycerinmonooleat zu dem Salz der C6-C12-Fettsäure oder deren Derivat mit einem aromatischen Substituenten und zu dem amphiphilen Blockcopolymer in der finalen Zusammensetzung 20:A:B beträgt, wobei 1 ≤ A ≤ 10 und 1 ≤ B ≤ 10, vorzugsweise 2 ≤ A ≤ 4 und 3 ≤ B ≤ 7, und noch bevorzugter 20:2:5 oder 20:4:5 beträgt.

7. Zusammensetzung nach einem der Ansprüche 1 bis 6, wobei die Konzentration des bioaktiven Makromoleküls mit Peptidbindungen in der finalen Zusammensetzung zwischen 0,01 mg/ml und 10,0 mg/ml, vorzugsweise zwischen 0,1 mg/ml und 5,0 mg/ml liegt.

8. Zusammensetzung nach einem der Ansprüche 1 bis 7, wobei das Gewichtsverhältnis von Glycerinmonooleat zu dem Salz der C6-C12-Fettsäure oder deren Derivat mit einem aromatischen Substituenten und zu dem amphiphilen Blockcopolymer und zu dem bioaktiven Makromolekül mit Peptidbindungen in der finalen Zusammensetzung 20:A:B:C beträgt, wobei 1 ≤ A ≤ 10, 1 ≤ B ≤ 10 und 0,01 ≤ C ≤ 10, vorzugsweise 2 ≤ A ≤ 4, 3 ≤ B ≤ 7 und 0,1 ≤ C ≤ 5, und noch bevorzugter 20:2:5:0,5 oder 20:2:5:1 oder 20:4:5:0,5 oder 20:4:5:1 beträgt.

9. Die Zusammensetzung gemäß einem der Ansprüche 1 bis 8, die mit pharmazeutisch akzeptablen Hilfsstoffen für die orale, intranasale, subkutane, intraperitoneale, intravenöse oder topische Verabreichung, vorzugsweise für die orale Verabreichung, formuliert ist.

## Revendications

1. Composition sous forme de nanoparticules cristallines liquides lyotropes comprenant du monooléate de glycérol, un sel d'un acide gras en C6-C12 ou son dérivé avec un substituant aromatique, un copolymère séquencé amphiphile de formule chimique H(OCH₂CH₂)ₐ(OCH(CH₃)CH₂)_{b}(OCH₂CH₂)ₐOH, dans laquelle 2 ≤ a ≤ 150 et 15 ≤ b ≤ 70, et une macromolécule bioactive comportant des liaisons peptidiques.

2. Composition selon la revendication 1, dans laquelle le sel de l'acide gras en C6-C12 est choisi parmi les sels alcalins de l'acide octanoïque, décanoïque ou dodé-canoïque, et son dérivé est choisi parmi les sels alcalins de l'acide 8-((2-hydroxy-benzoyl)amino)octanoïque, de préférence le sel de sodium de l'acide octanoïque et de l'acide 8-((2-hydroxybenzoyl)amino)octanoïque, plus préférablement le sel de sodium de l'acide 8-((2-hydroxybenzoyl)amino)octandique.

3. Composition selon l'une quelconque des revendications 1 et 2, dans laquelle le copolymère séquencé amphiphile de formule chimique H(OCH₂CH₂)ₐ(OCH(CH₃)CH₂)_{b}(OCH₂CH₂)ₐOH, dans laquelle, 2 ≤ a ≤ 150 et 15 ≤ b ≤ 70, est de préférence choisi parmi ceux dans lesquels 60 ≤ a ≤ 150 et 25 ≤ b ≤ 70, plus préférablement, est choisi parmi ceux dans lesquels 95 ≤ a ≤ 105 et 55 ≤ b ≤ 70.

4. Composition selon l'une quelconque des revendications 1 à 3, dans laquelle la macromolécule bioactive comportant des liaisons peptidiques est choisie parmi les enzymes, les protéines et les hormones, de préférence parmi le lysozyme, l'albumine sérique bovine, l'insuline, la calcitonine, l'octréotide, le liraglutide, le sémaglutide et le dulaglutide.

5. Composition selon l'une quelconque des revendications 1 à 4, dans laquelle la concentration en monooléate de glycérol dans la composition, est de 10 mg/ml à 100 mg/ml, de préférence de 20 mg/ml à 80 mg/ml, plus préférablement est de 20 mg/ml.

6. Composition selon l'une quelconque des revendications 1 à 5, dans laquelle le rapport pondéral entre le monooléate de glycérol, le sel d'acide gras en C6-C12 ou son dérivé avec un substituant aromatique et le copolymère séquencé amphiphile, dans la composition finale, est de 20:A:B, dans lequel 1 ≤ A ≤ 10 et 1 ≤ B ≤ 10, de préférence 2 ≤ A ≤ 4 et 3 ≤ B ≤ 7, plus préférablement est de 20:2:5 ou 20:4:5.

7. Composition selon l'une quelconque des revendications 1 à 6, dans laquelle la concentration de la macromolécule bioactive comportant des liaisons peptidiques est comprise entre 0,01 mg/ml et 10,0 mg/ml dans la composition finale, de préférence entre 0,1 mg/ml et 5,0 mg/ml dans la composition finale.

8. Composition selon l'une quelconque des revendications 1 à 7, dans laquelle le rapport pondéral entre le monooléate de glycérol, le sel d'acide gras en C6-C12 ou son dérivé avec un substituant aromatique, le copolymère séquencé amphiphile et la macromolécule bioactive comportant des liaisons peptidiques, dans la composition finale, est de 20:A:B:C, dans lequel 1 ≤ A ≤ 10, 1 ≤ B ≤ 10 et 0,01 ≤ C ≤ 10, de préférence dans lequel 2 ≤ A ≤ 4, 3 ≤ B ≤ 7 et 0,1 ≤ C ≤ 5, plus préférablement est de 20:2:5:0,5 ou 20:2:5:1 ou 20:4:5:0,5 ou 20:4:5:1.

9. Composition selon l'une quelconque des revendications 1 à 8, qui est formulée avec des excipients pharmaceutiquement acceptables pour une administration orale, intranasale, sous-cutanée, intrapéritonéale, intraveineuse ou topique, de préférence pour une administration orale.
